# FASCICULE DE BREVET EUROPEEN

(11) **EP 1 687 395 B1**
(45) Date de publication et mention de la délivrance du brevet: **14.03.2012**
(21) Numéro de dépôt: 04805559.4
(22) Date de dépôt: 26.11.2004
(51) Int. Cl.: C12M 1/22, C12M 3/04

(54) **BOITES BIOACTIVES POUR CULTURES CELLULAIRES**
BIOAKTIVE BEHÄLTER FÜR ZELLKULTUREN
BIOACTIVE BOXES FOR CELLULAR CULTURES

(30) Priorité: 28.11.2003 FR 0313993
(43) Date de publication de la demande: 09.08.2006
(73) Titulaire: CENTRE NATIONAL DE LA RECHERCHE SCIENTIFIQUE, 75794 Paris Cedex 16 (FR); Centre de Transfert de Technologie du Mans, 72000 Le Mans (FR); UNIVERSITE DE TECHNOLOGIE DE COMPIEGNE, 60200 Compiègne (FR)
(72) Inventeur: NAGEL, Marie-Danielle, 51450 Betheny (FR); LEGEAY, Gilbert, 72650 Saint Saturnin (FR)
(74) Mandataire: Grosset-Fournier, Chantal Catherine
(86) Numéro de dépôt international: PCT/FR2004/003031
(87) Numéro de publication internationale: WO 2005/054424

(56) Documents cités:
- WO-A-02/18448
- WO-A-03/095603
- DE-A- 3 743 136
- US-A- 4 036 693
- US-B1- 6 316 215
- DATABASE WPI Week 198838 Derwent Publications Ltd., London, GB; AN 1988-268191 XP002293616 & JP 63 196274 A (SUMITOMO ELECTRIC IND CO) 15 août 1988 (1988-08-15)
- PATENT ABSTRACTS OF JAPAN vol. 013, no. 302 (C-616), 12 juillet 1989 (1989-07-12) & JP 01 091770 A (IDEMITSU KOSAN CO LTD), 11 avril 1989 (1989-04-11)

## Description

La présente invention a pour objet des boîtes bioactives pour cultures cellulaires, et leurs utilisations pour la mise en oeuvre de procédés d'étude du vieillissement cellulaire, de la différenciation cellulaire, et de l'apoptose, de procédés de criblage de molécules anti-âge, de procédés de criblage de molécules antitumorales, de méthodes de diagnostic *in vitro* de la malignité de cellules tumorales et donc de méthodes de pronostic *in vitro* de tumeurs, ou de procédés d'études concernant des recherches sur la signalisation régulant la morphologie, la bioadhésion, la prolifération cellulaire et la communication intercellulaire.

Les travaux des Inventeurs ont initialement porté sur l'utilisation de membranes de cuprophane qui étaient plaquées sur le fond des boîtes de Pétri, puis ont testé les revêtements PVP (polyvinylpyrrolidone), HEC (hydroxyéthylcellulose), HPMC (hydroxypropylméthylcellulose), et CMC (carboxyméthylcellulose). Les avantages et inconvénients de ces différents matériaux sont résumés ci-après.

### 1) la membrane de cuprophane

Principales caractéristiques :
- Stabilité : 48 heures
- Morphologie cellulaire agrégée (formation d'agrégats cellulaires) et/ou arrondie
- Convient aux études de signalisation cellulaire à 45, 90 et 180 minutes (Faucheux et al., 1998, 1999, 2000, 2001, et 2002, et Duval et al., 1999)
- Prolifération cellulaire réduite
- Inductrice d'apoptose (notamment sur fibroblastes Swiss 3T3)

Principaux inconvénients :
- difficile à utiliser (plis),
- impossibilité de transfert industriel,
- n'est plus fabriquée commercialement.

### 2) revêtement de PVP

Principales caractéristiques :
- Morphologie cellulaire étalée (résultats identiques à ceux obtenus sur le témoin polystyrène (PS))

Ce revêtement est donc inutilisable.

### 3) revêtement de HEC

Principales caractéristiques :
- Stabilité 48 heures
- Morphologie cellulaire agrégée
- Prolifération cellulaire légèrement réduite

Principaux inconvénients :
- difficulté de mise en oeuvre pour avoir un dépôt régulier sur le support

### 4) revêtement de HPMC

Principales caractéristiques :
- Stabilité : 48 heures
- Morphologie cellulaire agrégée
- Prolifération cellulaire réduite
- Revêtement bien couvrant homogène et facile à mettre en oeuvre
- Revêtement utilisable pour l'injection de cellules agrégées à 90 minutes (cuprophane défavorable en raison de plis) ; ex : étude de la fonctionnalité de jonctions communicantes par l'injection de jaune de lucifer.

Son principal inconvénient réside dans le fait qu'il ne permet pas l'activation des cellules au delà de quelques heures.

### 5) revêtement de CMC

Principales caractéristiques :
- Stabilité : 48 heures et plus
- Morphologie cellulaire agrégée et/ou arrondie
- Prolifération cellulaire réduite.

Inconvénient: recouvrement incomplet du support : un anneau de PS reste accessible aux cellules à la périphérie (mise en oeuvre assez difficile, peu industrialisable).

La présente invention découle de la mise en évidence par les Inventeurs du fait que la bi-couche HPMC (ou alcool polyvinylique (PVA))-CMC permet de pallier aux inconvénients des revêtements étudiés ci-dessus.

Les avantages de la bicouche HPMC ou PVA sur CMC sont les suivants:
- Revêtement parfaitement couvrant et facile à fabriquer
- Stabilité : au moins 5 jours
- Stérilisable
- Morphologie cellulaire agrégée (fibroblastes Swiss 3T3, L929, mélanomes, lignée d'osteoblastes-like MC 3T3 E14 et MC 3T3 E1 24) ou arrondie (fibroblastes humains)
- Prolifération cellulaire réduite (mélanomes B16C3, B16F0, B16F10), MC 3T3, fibroblastes murins Swiss 3T3 et L 929.
- Différenciation des cellules témoignée par la synthèse de mélanine et une activité tyrosinase augmentée pour les 3 lignées de mélanomes
- Apoptose induite à 24 et 48 heures : activité caspase 3 augmentée à 24 et 48 heures et activité caspase 9 augmentée à 6 heures pour les fibroblastes Swiss 3T3.

La présente invention a pour objet des boîtes bioactives pour cultures cellulaires comportant sur leur fond une bicouche comprenant une couche primaire interne d'hydroxypropylméthylcellulose (HPMC), ou d'alcool polyvinylique (PVA), en contact avec le fond des boîtes, et une couche bioactive externe de carboxyméthylcellulose située sur ladite couche interne.

Les boîtes bioactives susmentionnées de l'invention sont davantage caractérisées en ce qu'elles se présentent sous forme de boîtes de pétri, telles que les boîtes de pétri en polystyrène d'origine commerciale, ou sous forme de plaques à puits multiples, au fond desquels se situe la bicouche.

Avantageusement, les boîtes bioactives susmentionnées de l'invention sont caractérisées en ce que les épaisseurs de la couche interne de HPMC ou de PVA, et de la couche externe de CMC, sont de quelques microns, notamment d'environ 1 à 5 microns.

L'invention concerne également un procédé de préparation de boîtes bioactives telles que définies ci-dessus, caractérisé en ce qu'il comprend :
- une étape d'activation de la surface du fond des boîtes par des décharges électromagnétiques,
- le dépôt de la couche interne de HPMC sur le fond des boîtes, puis séchage,
- le dépôt de la couche bioactive externe sur la couche primaire séchée obtenue à l'étape précédente, puis séchage.

L'invention a également pour objet l'utilisation de boîtes bioactives telles que définies ci-dessus, pour la mise en oeuvre :
- de procédés d'étude du vieillissement cellulaire, de la différenciation cellulaire, et de l'apoptose,
- de procédés de criblage de molécules anti-âge destinées à prévenir et retarder les effets du vieillissement,
- de procédés de criblage de molécules antitumorales destinées au traitement des cancers (recherche d'un effet potentialisateur, ou cumulatif par rapport à l'effet inducteur du revêtement),
- de méthodes de diagnostic *in vitro* de la malignité de cellules tumorales par mesure de la capacité résiduelle de cellules cancéreuses à se différencier, et à entrer en apoptose et donc de méthodes de pronostic *in vitro* de tumeurs,
- ou de procédés d'études concernant des recherches sur la signalisation régulant la morphologie, la bioadhésion, la prolifération cellulaires et la communication intercellulaire.

L'invention a plus particulièrement pour objet un procédé d'étude du vieillissement cellulaire, de la différenciation cellulaire, et de l'apoptose, caractérisé en ce qu'il comprend :
- une étape de mise en culture des cellules à étudier sur les boîtes définies ci-dessus,
- l'observation des cellules par microscope pour étudier leur morphologie,
- et/ou la détection, voire la quantification, d'une différenciation cellulaire, par mesure de la prolifération cellulaire, des protéines synthétisées, des marqueurs membranaires spécifiques exprimés,
- et/ou la détection, voire la quantification, de l'apoptose des cellules, par mesure de la viabilité (test d'exclusion au bleu trypan), de l'activation des caspases (méthodes fluorométriques ou colorimétriques, western blotting), de la fragmentation de la chromatine (essai TUNEL), ou de la formation de corps apoptotiques (coloration de Hoechst).

L'invention a également pour objet un procédé de criblage de molécules anti-âge destinées à prévenir et retarder les effets du vieillissement, caractérisé en ce qu'il comprend :
- une étape de mise en culture de cellules, telles que des fibroblastes, en présence des molécules anti-âge à étudier, sur les boîtes de culture définies ci-dessus,
- l'observation des cellules par microscope pour étudier leur morphologie,
- et/ou la détection, voire la quantification, de la prolifération, et de synthèses, notamment de protéines cellulaires telles que le collagène,
- et la comparaison avec les observations et résultats obtenus sur des cultures de cellules utilisées en tant que témoins, lesdites cultures témoins étant effectuées par mise en culture desdites cellules en l'absence desdites molécules anti-âge à étudier, sur les boîtes définies ci-dessus.

L'invention concerne également un procédé de criblage de molécules antitumorales destinées au traitement des cancers, caractérisé en ce qu'il comprend :
- une étape de mise en culture de cellules, telles que des cellules de mélanome animales ou humaines, en présence des molécules antitumorales à étudier, sur les boîtes de culture définies ci-dessus,
- l'observation des cellules par microscope pour étudier leur morphologie et leur différenciation (mélanogenèse),
- et/ou la détection, voire la quantification, de leur prolifération, différenciation (détection de synthèse de mélanine et activité tyrosinase, synthèse de fibronectine, de l'expression de marqueurs tumoraux comme MelCam, l'expression de cadhérines, (mise en évidence par western blotting par ex.) et de l'apoptose (étude des activités caspases par spectrofluorométrie, western blotting par ex. et autres méthodes telles que TUNEL, coloration de Hoechst...)
- et la comparaison avec les observations et résultats obtenus sur des cultures de cellules utilisées en tant que témoins, lesdites cultures témoins étant effectuées par mise en culture desdites cellules en l'absence desdites molécules antitumorales à étudier, sur les boîtes de culture définies ci-dessus.

L'invention a également pour objet une méthode de diagnostic *in vitro* de la malignité de cellules tumorales par mesure de la capacité résiduelle de cellules cancéreuses à se différencier, caractérisée en ce qu'elle comprend :
- une étape de mise en culture de cellules cancéreuses, telles que des cellules de mélanomes humains obtenues à partir de biopsies, sur les boîtes de culture définies ci-dessus,
- l'observation des cellules par microscope pour étudier leur morphologie et différenciation, et/ou la détection, voire la quantification, de leur prolifération (détection de synthèse de mélanine intracellulaire selon la technique décrite par De Pauw-Gillet et al (Anticancer Research 1990, 10, 391-96), d'activité tyrosinase (méthode de dosage utilisée décrite par Steinberg et al (J Cell. Physiol. 1976, 87, 265-76), de fibronectine, (immunocytochimie, RT-PCR)), de leur viabilité, et de l'apoptose.

L'invention concerne également l'application de la méthode de diagnostic susmentionnée au pronostic *in vitro* de tumeurs.

L'invention a également pour objet des procédés d'études concernant des recherches de signalisation cellulaire (voies de l'AMPc et des MAPK en particulier), sur la morphologie, la bioadhésion (par rapport à l'état d'activation ou d'inactivation des intégrines), la prolifération, la communication intercellulaire (détection de cadhérines, connexines, intégrines), à partir de techniques diverses telles que observations microscopiques, western blotting, RT-PCR, cytométrie en flux, immunomarquages, injection de jaune de lucifer dans les agrégats, lesdits procédés étant effectués par mise en culture des cellules à étudier sur des boîtes de culture définies ci-dessus, observation et mesure des évènements susmentionnés.

L'invention sera davantage illustrée à l'aide de la description détaillée qui suit d'un procédé d'obtention d'une boîte de culture comportant une bicouche telle que définie ci-dessus selon l'invention, ainsi que de leur utilisation dans le cadre de la mise en culture de cellules.

### I) Description expérimentale de réalisation des revêtements de boites de pétri

Les travaux sont effectués avec des boîtes d'origine commerciale, généralement en polystyrène. Elles peuvent être de petite, moyenne ou grande taille. Ce peut également être des systèmes à puits multiples

Les travaux ont été effectués avec des boites en provenance de Greiner^{®} Bio one.

Les boîtes sont utilisées en l'état (pas de lavage qui risque de polluer ou altérer l'état de surface).

Les traitements s'effectuent en plusieurs étapes lesquelles sont effectuées en milieu "salle propre ou salle blanche".

### 1) activation de surface par des décharges électromagnétiques, (plasma, décharges couronnes).

Par exemple par plasma on utilise un système RF 13,56 MHz, avec un réacteur d'environ 20 litres de capacité. On procède la façon suivante :

Les objets sont placés à l'intérieur du réacteur, puis on applique le vide (jusqu'à environ 1 Pa), puis on fait rentrer du gaz, par exemple de l'oxygène jusqu'à une pression partielle de 5 à 10 Pa. La décharge électromagnétique est allumée et maintenue (c'est automatique) à une certaine puissance (par exemple 50W) et pendant 5 minutes.

A l'issue de ce temps les boites sont récupérées. L'efficacité du traitement est appréciée par mesure du mouillage avec le test de la goutte d'eau (l'angle de contact est inférieur à 25°).

### 2) dépôt d'une couche (primaire)

Dans les minutes suivant l'activation de surface on dépose une couche du polymère HPMC E4M à partir d'une solution aqueuse , que l'on aura dégazée pour chasser l'air dissout, à 0,2%/eppi. Les boîtes sont remplies puis vidées après quelques instants, mises à égoutter en milieu aéré puis séchées en étuve ventilée à 50°C pendant 1heure environ.

L'efficacité du traitement est appréciée par mesure du mouillage avec le test de la goutte d'eau : l'angle de contact est de l'ordre de 50à 60°

### 3) Dépôt d'une couche bioactive

La deuxième couche de polymère CMC 7LF est déposée comme précédemment à partir d'une solution aqueuse (eau ppi) préalablement dégazée, et à une concentration de 0,2%.

Après égouttage et séchage à 50°C les boîtes sont emballées soit individuellement soit par lot de 10 et sous un courant d'azote.

Pour le contrôle qualité l'efficacité du traitement est appréciée par mesure du mouillage avec le test de la goutte d'eau : l'angle de contact est de l'ordre de 40 à 45°

Dans la suite, les boîtes ainsi obtenues seront notamment désignées boîtes enduites de cellulose ou Boîtes pour Culture cellulaire Bioactives (BCB)

### II) ASPECTS BIOLOGIQUES

### 1) Préparation des boîtes enduites de cellulose

Avant utilisation, les boîtes enduites de cellulose sont traitées pendant 1 heure à T du laboratoire avec une solution d'antibiotiques (pénicilline, streptomycine) à 1% en eau ultra-pure. Elles sont ensuite rincées 3 fois à l'eau ultra-pure.

Récemment, les solutions d'HPMC et de CMC ont été filtrées stérilement avant d'être utilisées pour l'enduction. Les boîtes ont alors seulement été soumises à un bain d'eau ultra-pure pendant 1 heure à T du laboratoire avant d'être ensemencées avec les cellules.

Après stérilisation des boîtes pour culture cellulaire bioactives (BCB) par l'oxyde d'éthylène et désorption du gaz selon les méthodes lente ou accélérée classiquement utilisées dans l'industrie, les agrégats cellulaires peuvent être maintenus en culture pendant une semaine si l'on rajoute du milieu complet au 3^{ème} ou 4^{ème} jour de la culture.

### 2) Caractérisation biologique du revêtement

Après le traitement habituel suivi d'1 heure d'incubation à 37°C en présence d'une solution de fibronectine (Fn) humaine ou bovine à une concentration de 3 µg/cm² et 3 lavages en eau ultra-pure, les boîtes sont ensemencées avec des cellules et la morphologie des ces dernières est observée après 3 heures. L'absence d'étalement cellulaire indique l'absence d'adsorption de Fn par la surface cellulosique.

Cette observation est confirmée par l'absence de marquage positif si, après avoir été incubée avec du sérum, ou avec des cellules de mélanome sécrétant de la Fn, la boîte enduite de cellulose est soumise à un immunomarquage indirect (anticorps primaire de lapin réagissant avec la Fn bovine et la Fn murine, anticorps secondaire de chèvre anti-lapin couplé à la rhodamine). Dans les mêmes situations, un résultat positif est obtenu avec le contrôle PS.

### 3) Effet inducteur sur les cellules adhérentes : contrôle polystyrène (PS) pour culture de cellules

Les cellules sont cultivées en milieu supplémenté par 10% de sérum de veau foetal, 1% de L-Glutamine et 0.5% d'antibiotiques (pénicilline, streptomycine).

### -a-morphologie cellulaire : celle-ci est observée au microscope inversé en contraste de phase, ou au microscope électronique environnemental (ESEM).

Des cellules arrondies et agrégées (étalées sur PS) : lignées murines : fibroblastes Swiss 3T3, L929, Mélanomes B16 C3, B16 F0, B16 F10, osteoblastes MC 3T3 E1 sous clones 4 et 24, cellules primaires de métastases de mélanomes B16F10 induites chez la souris C57 Black 6 sont utilisées.

L'agrégation des cellules de mélanomes B16 est dépendante du calcium : en l'absence de Ca⁺⁺ (milieu sans sérum, monencine, tétrandrine), le pourcentage de cellules isolées est très significativement augmenté pour les 3 lignées après 1 et 3 heures.

### -b-prolifération : 10 000 cellules sont ensemencées par cm². Les cellules sont comptées (hemocytomètre de Malassez) après 24 et 48 heures de culture.

A 24 heures, les différences éventuellement observées entre le contrôle PS et le revêtement cellulosique ne sont pas significatives.

Par contre, à 48 heures, les cellules sur cellulose sont 2 fois moins nombreuses que celles sur PS.

Ceci a été vérifié avec toutes les lignées étudiées.

Il est à noter que les lignées B16 F0 et B16 F10 prolifèrent plus sur cellulose que la lignée B16 C3, non métastatique. Le nombre de cellules B16 F0 et F10 est multiplié par 3 sur cellulose. Il est multiplié par 6 sur PS, en présence ou en absence de MSH 10⁻⁷ M melanin stimulating hormon utilisée comme deuxième contrôle. Le nombre de cellules B16 C3 reste stable après 48 heures sur cellulose, il est multiplié par 2 sur PS en présence comme en absence de MSH.

Des cultures en milieu semi-solide d'explants de métastases induites chez la souris par injection de cellules du mélanome B16F10 (selon la technique décrite par Duval et al, : 1999. Cell & Materials, 9, 31-42) ont montré, après 14 jours au contact d'échantillons BCB, une inhibition très significative de la prolifération des cellules autour de l'explant, comparativement aux voiles cellulaires largement développés au contact des échantillons de PS contrôles.

### -c-viabilité : test d'exclusion au bleu trypan

Après 48 heures de culture, la viabilité des cellules rondes et agrégées sur cellulose est inférieure à celle mesurée sur PS (environ 94% sur cellulose et 99% sur PS).

La viabilité des ostéoblastes MC3T3 E1 est très affectée sur BCB. 50% des cellules initialement ensemencées ont disparu à 24 heures, et 75% à 48 heures. (cf f-apoptose).

### -d-synthèses :

Protéines totales par cellule (dosage par la méthode de Bradford, kit commercial) : les lignées Swiss 3T3 et B16 montrent une synthèse augmentée après 48 heures sur cellulose. Les autres lignées n'ont pas été étudiées.

Synthèse de Fn : les cellules agrégées sur cellulose présentent un marquage périphérique positif dès 3 heures, augmenté à 24 heures (immunomarquage indirect utilisant un anticorps primaire anti Fn de souris, non réactif vis à vis de la Fn bovine). MSH favorise aussi une augmentation de synthèse de Fn qui apparaît bien localisée au niveau des contacts cellulaires pour les trois lignées de mélanomes cultivées sur PS.

Synthèse de mélanine intracellulaire : selon la technique décrite par De Pauw-Gillet et al (Anticancer Research 1990, 10, 391-96).

Une augmentation significative de la mélanine intracellulaire est observée après 48 heures pour les 3 lignées B16 agrégées sur cellulose. L'effet inducteur du revêtement cellulosique sur la synthèse de mélanine est au moins équivalent sinon supérieur à celui obtenu avec MSH 10⁻⁷ M

Ce résultat est complété et confirmé par une augmentation de l'activité tyrosinase dans les 3 lignées cultivées sur cellulose à 48 heures. La méthode de dosage utilisée est celle décrite par Steinberg et al (J Cell. Physiol. 1976, 87, 265-76). Là encore, les résultats sont équivalents ou supérieurs à ceux mesurés en présence de MSH.

Des cultures en milieu semi-solide d'explants de métastases induites chez la souris par injection de cellules B16F10 ont montré, après 14 jours au contact d'échantillons BCB, une charge importante en mélanine des cellules autour de l'explant, comparativement aux voiles cellulaires amélaniques largement développés au contact des échantillons de PS contrôles.

### -e-expression de protéines membranaires : récepteurs et marqueurs

Par immunomarquages et western blotting, les cadhérines N ont été évaluées. Les cellules agrégées sur cellulose montrent une augmentation des cadhérines N. Toutefois, une décroissance des β-caténines (immunoprécipitation et western blotting) suggèrent une perte de fonctionnalité des cadhérines N exprimées sur cellulose.

Le marqueur tumoral Mel-Cam, observable à partir de cellules B16 F10 (western blots) cultivées 48 heures sur PS n'est plus exprimé à la surface des cellules cultivées le même temps sur cellulose.

### -f-apoptose : déterminée par mesure d'activité de la caspase 3 par fluorométrie ou western blotting, et méthode TUNEL (kit commercial)

L'activité caspase 3 est généralement augmentée à 24 et 48 heures dans les cellules agrégées sur cellulose. Ce résultat est confirmé par l'essai TUNEL. Les cellules testées sont : les fibroblastes Swiss 3T3, les ostéoblastes MC 3T3 E1, et les 3 lignées de mélanomes B16. Les autres lignées et les cultures primaires de métastases induites par injection de cellules B16 F10 chez la souris seront par la suite vérifiées.

Des essais complémentaires par marquage à l'Annexine V en cytométrie de flux ont montré une survie prolongée des cellules de mélanomes B16 à 5 et 8 jours et même au-delà pour les B16C3. Au contraire, les ostéoblastes de lignée MC3T3 E1 entrent très précocement en apoptose (avant 24 heures de culture).

Les BCB mettent donc en évidence des sensibilités différentes à l'apoptose suivant les types cellulaires analysés.

Des explants de métastases induites chez la souris par injection de cellules B16F10, cultivés 14 jours sur milieu semi-solide, ont montré au contact d'échantillons BCB un pourcentage de cellules apoptotiques significativement plus élevé que celui établi à partir des contrôles cultivés au contact de PS (méthodes utilisées : marquage à l'Annexine V en cytométrie de flux et TUNEL).

### Conclusions

L'effet inducteur est un effet différenciant : cf paragraphes b, d, et e, ci-dessus.

Le matériau a une efficacité égale ou supérieure à MSH 10⁻⁷ M, agent différenciant utilisé comme contrôle dans les cultures sur PS.

Le revêtement est inducteur d'apoptose chez des cellules normales et cancéreuses : cf paragraphes c, et f, ci-dessus.

Essai de culture prolongée : 5 jours, réalisé avec des B16 C3.

Résultats concluants : gros amas très mélaniques, maintien de l'état agrégé.

### Références bibliographiques :

- FAUCHEUX, N., WAROCQUIER-CLEROUT, R., HAYE, B., NAGEL, M-D. : 1998. "Cyclic AMP in cells adhering to bioincompatible (cuprophan) and biocompatible (AN 69) substrates." J. Biomed. Mat. Res., 39, 506-510.
- DUVAL, J-L., FAUCHEUX, N., WAROCQUIER-CLEROUT, R., NAGEL, M-D. : 1999. "Study of melanoma cell behavior in cell and organ cultures in vitro : use of biomaterials as potential tools of cell activation." Cell & Materials, 9, 31-42.
- FAUCHEUX, N., WAROCQUIER-CLEROUT, R., DUVAL, J-L., HAYE, B., NAGEL, M-D. : 1999. "cAMP levels in cells attached to AN 69 and Cuprophan : cAMP dependence of cell aggregation and the influence of serum." Biomaterials, 20, 159-165.
- FAUCHEUX, N., HAYE, B., NAGEL, M-D. : 2000. "Activation of cyclic AMP in cells adhering to biomaterials : regulation by vitronectin and fibronectin-integrin binding." Biomaterials, 21, 1031-1038.
- FAUCHEUX, N., DUFRESNE, M., NAGEL, M-D. : 2002. "Organisation of cyclic AMP-dependent connexin 43 in Swiss 3T3 cells attached to a cellulose substratum." Biomaterials, 23, 413-421.
- FAUCHEUX, N., CORREZE, C., HAYE, B., NAGEL, M-D. : 2001. "Accumulation of cyclic AMP in Swiss 3T3 cells adhering to a cellulose biomaterial substratum through interaction with adenylyl cyclase." Biomaterials, 22, 2993-2998.
- FAUCHEUX N., NAGEL M-D. : 2002. "Cyclic AMP - dependent aggregation of Swiss 3T3 cells on a cellulose substratum (Cuprophan) and decreased cell membrane Rho A." Biomaterials, 23, 2295-2301.
- FAUCHEUX N., ZAHM JM., BONNET N., LEGEAY G., NAGEL M-D. : 2004 "Gap junction communication between cells aggregated on a cellulose-coated polystyrene : influence of connexin 43 phosphorylation." Biomaterials, 25, 2501-2506.
- GEKAS J., HINDIE M., FAUCHEUX N., LANVIN O., MAZIÈRE C., FUENTES V., GOUILLEUX-GRUART V., DAVID B., MAZIERE JC., LASSOUED K., NAGEL MD. : 2004. "The inhibition of cell spreading on a cellulose substrate (Cuprophan) induces an apoptotic process via a mitochondria-dependent pathway." FEBS Lett., 563, 103-107.

## Revendications

1. Boîtes bioactives pour cultures cellulaires comportant sur leur fond une bicouche comprenant une couche primaire interne d'hydroxypropylméthylcellulose (HPMC), ou d'alcool polyvinylique (PVA), en contact avec le fond des boîtes, et une couche bioactive externe de carboxyméthylcellulose (CMC) située sur ladite couche interne.

2. Boîtes bioactives selon la revendication 1, **caractérisées en ce qu'**elles se présentent sous forme de boîtes de pétri, telles que les boîtes de pétri en polystyrène d'origine commerciale, ou sous forme de plaques à puits multiples, au fond desquels se situe la bicouche.

3. Boîtes bioactives selon la revendication 1 ou 2, **caractérisées en ce que** les épaisseurs de la couche interne de HPMC ou de PVA, et de la couche externe de CMC, sont de quelques microns, notamment d'environ 1 à 5 microns.

4. Procédé de préparation de boîtes bioactives selon l'une des revendications 1 à 3, **caractérisé en ce qu'**il comprend :
- une étape d'activation de la surface du fond des boîtes par des décharges électromagnétiques,
- le dépôt de la couche interne de HPMC sur le fond des boîtes, puis séchage,
- le dépôt de la couche bioactive externe sur la couche primaire séchée obtenue à l'étape précédente, puis séchage.

5. Utilisation de boîtes bioactives selon l'une des revendications 1 à 3, pour la mise en oeuvre :
- de procédés d'étude du vieillissement cellulaire, de la différenciation cellulaire, et de l'apoptose,
- de procédés de criblage de molécules anti-âge destinées à prévenir et retarder les effets du vieillissement,
- de procédés de criblage de molécules antitumorales destinées au traitement des cancers,
- de méthodes de diagnostic *in vitro* de la malignité de cellules tumorales par mesure de la capacité résiduelle de cellules cancéreuses à se différencier, et à entrer en apoptose et donc de méthodes de pronostic *in vitro* de tumeurs,
- ou de procédés d'études concernant des recherches sur la signalisation régulant la morphologie, la bioadhésion, la prolifération cellulaires et la communication intercellulaire.

6. Procédé d'étude du vieillissement cellulaire, de la différenciation cellulaire, et de l'apoptose, **caractérisé en ce qu'**il comprend :
- une étape de mise en culture des cellules à étudier sur les boîtes définies dans l'une des revendications 1 à 3,
- l'observation des cellules par microscope pour étudier leur morphologie,
- et/ou la détection, voire la quantification, d'une différenciation cellulaire, par mesure de la prolifération cellulaire, des protéines synthétisées, des marqueurs membranaires spécifiques exprimés,
- et/ou la détection, voire la quantification, de l'apoptose des cellules, par mesure de la viabilité, de l'activation des caspases, de la fragmentation de la chromatine ou de la formation de corps apoptotiques

7. Procédé de criblage de molécules anti-âge destinées à prévenir et retarder les effets du vieillissement, **caractérisé en ce qu'**il comprend :
- une étape de mise en culture de cellules, telles que des fibroblastes, en présence des molécules anti-âge à étudier, sur les boîtes de pétri définies dans l'une des revendications 1 à 3,
- l'observation des cellules par microscope pour étudier leur morphologie,
- et/ou la détection, voire la quantification, de la prolifération et de synthèses,
- et la comparaison avec les observations et résultats obtenus sur des cultures de cellules utilisées en tant que témoins, lesdites cultures témoins étant effectuées par mise en culture desdites cellules en l'absence desdites molécules anti-âge à étudier, sur les boîtes définies dans l'une des revendications 1 à 3.

8. Procédé de criblage de molécules antitumorales destinées au traitement des cancers, **caractérisé en ce qu'**il comprend :
- une étape de mise en culture de cellules, telles que des cellules de mélanome animales ou humaines, en présence des molécules antitumorales à étudier, sur les boîtes définies dans l'une des revendications 1 à 3,
- l'observation des cellules par microscope pour étudier leur morphologie et leur différenciation,
- et/ou la détection, voire la quantification, de leur prolifération, différenciation, et de l'apoptose,
- et la comparaison avec les observations et résultats obtenus sur des cultures de cellules utilisées en tant que témoins, lesdites cultures témoins étant effectuées par mise en culture desdites cellules en l'absence desdites molécules antitumorales à étudier, sur les boîtes définies dans l'une des revendications 1 à 3.

9. Méthode de diagnostic *in vitro* de la malignité de cellules tumorales par mesure de la capacité résiduelle de cellules cancéreuses à se différencier, **caractérisée en ce qu'**elle comprend :
- une étape de mise en culture de cellules cancéreuses, telles que des cellules de mélanomes humains obtenues à partir de biopsies, sur les boîtes de pétri définies dans l'une des revendications 1 à 3,
- l'observation des cellules par microscope pour étudier leur morphologie et différenciation, et/ou la détection, voire la quantification, de leur prolifération, viabilité et de l'apoptose.

10. Application de la méthode de diagnostic selon la revendication 9, au pronostic *in vitro* de tumeurs.

## Claims

1. Bioactive dishes for cell cultures comprising on their bottom a bilayer comprising an internal primary layer made of hydroxypropylmethylcellulose (HPMC), or polyvinyl alcohol (PVA) in contact with the bottom of the dishes, and an external bioactive layer made of carboxypropylmethylcellulose (CMC) situated on said internal layer.

2. Bioactive dishes according to claim 1, **characterized in that** they are presented in the form of Petri dishes, such as polystyrene Petri dishes of commercial origin, or in the form of multi-well plates, on the bottom of which the bilayer is situated.

3. Bioactive dishes according to claim 1 or 2, **characterized in that** the thicknesses of the internal HPMC or PVA layer, and of the external CMC layer, are a few microns, in particular approximately 1 to 5 microns.

4. Method for preparing bioactive dishes according to one of the claims 1 to 3, **characterized in that** it comprises:
- a stage of activation of the surface of the bottom of the dishes by electromagnetic discharges,
- the depositing of the internal HPMC layer on the bottom of the dishes, then drying,
- the depositing of the external bioactive layer on the dried primary layer obtained in the preceding stage, then drying.

5. Use of bioactive dishes according to one of the claims 1 to 3 for the implementation of:
- methods for studying cell ageing, cell differentiation, and apoptosis,
- methods for screening anti-ageing molecules intended to prevent and delay the effects of ageing,
- methods for screening antitumor molecules intended for the treatment of cancer,
- methods for *in vitro* diagnosis of tumor-cell malignancy by measurement of the residual ability of cancer cells to differentiate, and to enter into apoptosis and therefore methods for *in vitro* tumor prognosis,
- or study methods relating to research into signalling controlling morphology, bioadhesion, cell proliferation and intercellular communication.

6. Method for studying cell ageing, cell differentiation, and apoptosis, **characterized in that** it comprises:
- a stage of culturing cells to be studied in the dishes defined in one of claims 1 to 3,
- the observation of the cells by microscope in order to study their morphology,
- and/or the detection, or even the quantification, of cell differentiation, by measurement of cell proliferation, synthesized proteins, and specific membrane markers expressed,
- and/or the detection, or even the quantification, of the apoptosis of the cells, by measurement of viability, activation of the caspases, chromatin fragmentation, or formation of apoptotic bodies.

7. Method for screening anti-ageing molecules intended to prevent and delay the effects of ageing, **characterized in that** it comprises:
- a stage of culturing cells, such as fibroblasts, in the presence of the anti-ageing molecules to be studied, in the culture dishes defined in one of claims 1 to 3,
- the observation of the cells by microscope in order to study their morphology,
- and/or the detection, or even the quantification, of the proliferation and syntheses,
- and the comparison with the observations and results obtained on cultures of cells used as controls, said control cultures being carried out by culturing said cells in the absence of said anti-ageing molecules to be studied, in the dishes defined in one of claims 1 to 3.

8. Method for screening antitumor molecules intended for the treatment of cancer, **characterized in that** it comprises:
- a stage of culturing cells, such as animal or human melanoma cells, in the presence of the antitumor molecules to be studied, in the culture dishes defined in one of claims 1 to 3,
- observation of the cells by microscope in order to study their morphology and their differentiation,
- and/or the detection, or even the quantification, of their proliferation, differentiation and apoptosis,
- and comparison with the observations and results obtained on cell cultures used as controls, said control cultures being carried out by culturing said cells in the absence of said antitumor molecules to be studied, in the culture dishes defined in one of claims 1 to 3.

9. Method for *in vitro* diagnosis of the malignancy of tumor cells by measurement of the residual ability of cancer cells to differentiate, **characterized in that** it comprises:
- a stage of culturing cancer cells, such as human melanoma cells obtained from biopsies, in the culture dishes defined in one of claims 1 to 3,
- the observation of the cells by microscope in order to study their morphology and differentiation, and/or the detection, or even the quantification, of their proliferation viability and apoptosis.

10. Application of the diagnosis method according to claim 9 to the *in vitro* prognosis of tumors.

## Patentansprüche

1. Bioaktive Schalen für Zellkulturen, die auf ihrem Boden eine Doppelschicht enthalten, die eine primäre innere Schicht aus Hydroxypropylmethylcellulose (HPMC) oder Polyvinylalkohol (PVA) in Kontakt mit dem Boden der Schalen, und eine bioaktive äußere Schicht aus Carboxymethylcellulose (CMC), die auf der inneren Schicht liegt, umfasst.

2. Bioaktive Schalen nach Anspruch 1, **dadurch gekennzeichnet, dass** sie die Form von Petrischalen, wie etwa handelsüblichen Petrischalen aus Polystyrol, oder die Form von Multititerplatten aufweisen, auf deren Boden die Doppelschicht liegt.

3. Bioaktive Schalen nach Anspruch 1 oder 2, **dadurch gekennzeichnet, dass** die Dicken der inneren Schicht aus HPMC oder aus PVA und der äußeren Schicht aus CMC einige Mikron, insbesondere etwa 1 bis 5 Mikron, betragen.

4. Verfahren zur Herstellung von bioaktiven Schalen nach einem der Ansprüche 1 bis 3, **dadurch gekennzeichnet, dass** es umfasst:
- einen Schritt zum Aktivieren der Oberfläche des Bodens der Schalen durch elektromagnetische Entladungen,
- Ablagern der inneren Schicht aus HPMC auf dem Boden der Schalen, dann Trocknen,
- Ablagern der äußeren bioaktiven Schicht auf der getrockneten primären Schicht, die im vorhergehenden Schritt erhalten wurde, dann Trocknen.

5. Verwendung von bioaktiven Schalen nach einem der Ansprüche 1 bis 3 zur Durchführung:
- von Untersuchungsverfahren zur Zellalterung, zur Zelldifferenzierung und zur Apoptose,
- von Verfahren zum Screenen von Anti-Aging-Molekülen, die dazu bestimmt sind, den Auswirkungen der Alterung vorzubeugen und sie zu verzögern,
- von Verfahren zum Screenen von Antitumormolekülen, die zur Krebsbehandlung bestimmt sind,
- von Methoden zur *In-vitro*-Diagnose der Malignität von Tumorzellen durch Messen der Restkapazität der Krebszellen, sich zu differenzieren, und in die Apoptose einzutreten und daher von In-vitro-Prognosemethoden von Tumoren,
- oder von Untersuchungsverfahren, die Forschungen zur Signalgebung betreffen, die die Morphologie, die Bioadhäsion, die Zellproliferation und die interzelluläre Kommunikation reguliert.

6. Untersuchungsverfahren zur Zellalterung, zur Zelldifferenzierung und zur Apoptose, **dadurch gekennzeichnet, dass** es umfasst:
- einen Schritt zum Kultivieren der zu untersuchenden Zellen in den Schalen, die in einem der Ansprüche 1 bis 3 definiert wurden,
- Beobachten der Zellen durch das Mikroskop, um ihre Morphologie zu untersuchen,
- und/oder Nachweisen oder sogar Quantifizieren einer Zelldifferenzierung, durch Messen der Zellproliferation, synthetischer Proteine, spezifischer exprimierter Membranmarker,
- und/oder Nachweisen oder sogar Quantifizieren der Zellapoptose durch Messen der Lebensfähigkeit, der Caspasenaktivierung, der Chromatinfragmentierung oder der Bildung von apoptotischen Körpern.

7. Verfahren zum Screenen von Anti-Aging-Molekülen, die dazu bestimmt sind, den Auswirkungen der Alterung vorzubeugen und sie zu verzögern, **dadurch gekennzeichnet, dass** es umfasst:
- einen Schritt zum Kultivieren von Zellen, wie etwa Fibroblasten, in Gegenwart von zu untersuchenden Anti-Aging-Molekülen in Petrischalen, die in einem der Ansprüche 1 bis 3 definiert wurden,
- Beobachten der Zellen durch das Mikroskop, um ihre Morphologie zu untersuchen,
- und/oder Nachweisen oder sogar Quantifizieren von Proliferation und Synthesen,
- und Vergleichen mit Beobachtungen und Ergebnissen, die aus Zellkulturen erhalten wurden, die als Kontrollen verwendet wurden, wobei die Kontrollkulturen durch Kultivieren der Zellen in Abwesenheit der zu untersuchenden Anti-Aging-Moleküle durchgeführt wurden, in Schalen, die in einem der Ansprüche 1 bis 3 definiert wurden.

8. Verfahren zum Screenen von Antitumormolekülen, die zur Krebsbehandlung bestimmt sind, **dadurch gekennzeichnet, dass** es umfasst:
- einen Schritt zum Kultivieren von Zellen, wie etwa tierischen oder humanen Melanomzellen, in Gegenwart von zu untersuchenden Antitumormolekülen in Petrischalen, die in einem der Ansprüche 1 bis 3 definiert wurden,
- Beobachten der Zellen durch das Mikroskop, um ihre Morphologie und ihre Differenzierung zu untersuchen,
- und/oder Nachweisen oder sogar Quantifizieren von der Proliferation, der Differenzierung und der Apoptose,
- und Vergleichen mit Beobachtungen und Ergebnissen, die aus Zellkulturen erhalten wurden, die als Kontrollen verwendet wurden, wobei die Kontrollkulturen durch Kultivieren der Zellen in Abwesenheit der zu untersuchenden Antitumormoleküle durchgeführt wurden, in Schalen, die in einem der Ansprüche 1 bis 3 definiert wurden.

9. Methode zur *In-vitro*-Diagnose der Malignität der Tumorzellen durch Messen der Restkapazität der Krebszellen, sich zu differenzieren, **dadurch gekennzeichnet, dass** sie umfasst:
- einen Schritt zum Kultivieren von Krebszellen, wie etwa humanen Melanomzellen, die aus Biopsien erhalten wurden, in Petrischalen, die in einem der Ansprüche 1 bis 3 definiert wurden,
- Beobachten der Zellen durch das Mikroskop, um ihre Morphologie und Differenzierung zu untersuchen, und/oder Nachweisen oder sogar Quantifizieren ihrer Proliferation, Lebensfähigkeit und der Apoptose.

10. Anwenden der Diagnosemethode nach Anspruch 9 auf die *In-vitro*-Prognose von Tumoren.
